# EUROPEAN PATENT APPLICATION

(11) **EP 2 053 043 A1**
(43) Date of publication of application: **29.04.2009**
(21) Application number: 07380294.4
(22) Date of filing: 26.10.2007
(51) Int. Cl.: C07D 215/18, A61K 31/4704, A61P 11/06

(54) **Crystalline salt of montelukast**

(71) Applicant: INKE, S.A., 08755 Castellbisbal (Barcelona) (ES)
(72) Inventor: Huguet Clotet, Joan, 08970 Sant Joan Despi Barcelona (ES); Peirats Masia, Jordi, 08320 El Masnou Barcelona (ES)
(74) Representative: ABG Patentes, S.L.

(57) **Abstract**

The present invention refers to the novel cyclopropylamine salt of montelukast in crystalline form and its use in the process for the preparation of highly pure amorphous montelukast sodium.

## Description

### FIELD OF THE INVENTION

The present invention is directed to a novel crystalline salt of montelukast and to its use in the preparation of sodium montelukast and in the manufacture of a medicament.

### BACKGROUND

Montelukast or (1-{1-(R)-(E)-{3-[2-(7-Chloroquinolin-2-yl)-vinyl]-phenyl}-3-[2-(1-hydroxy-1-methylethyl)-phenyl]-propylsulfanylmethyl}-cyclopropyl)-acetic acid has the following formula (I) wherein R = H.

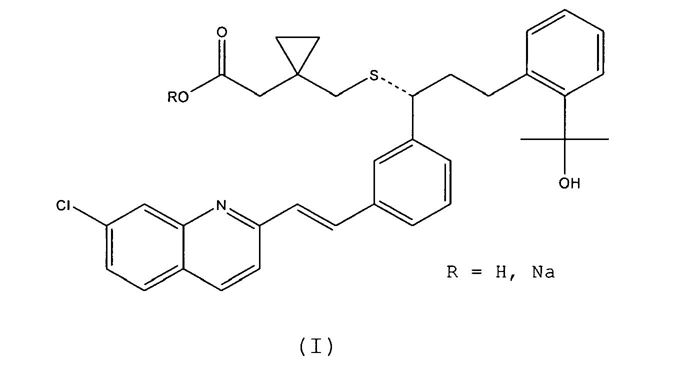

Sodium montelukast (R = Na in formula (I)) is used in the treatment of asthma. It is marketed under the name of SINGULAIR® (Merck) as oral tablets, chewable tablets and granules.

EP 0 480 717 B1 described for the first time a family of compounds wherein sodium montelukast was comprised. The synthesis described in said patent involved methyl esters such as methyl 2-[(3S)-[3-[(2E)-(7-chloroquinolyn-2-yl)ethenylphenyl]-3-hydroxipropyl]benzoate and comprised the coupling between methyl 1-(mercaptometyl)-cyclopropaneacetate and an appropriate mesylate produced *in situ*. The methyl ester of montelukast was hydrolyzed into its acid and directly transformed into its corresponding sodium salt (formula (I)). The tedious chromatographic purifications of the methyl esters and final products required, makes the above process unsuitable for large scale production. Additionally, the yields obtained are poor. Sodium montelukast, obtained by this method, is an oily substance. The product in amorphous form is obtained only after lyophilisation, another process that is not economical in a large scale production.

EP 0 737 186 B1 describes an improved process for the synthesis of sodium montelukast and dicyclohexylammonium montelukast, which differed from the process described in EP 0 480 717 B1 in the use of the dilithium salt of 1-(mercaptomethyl)cyclopropaneacetic acid, instead of the methyl ester for the coupling reaction with the mesylate. Said mesylate had the same formula as in EP 0 480 717 B1 but was added in its crystalline form. The process directly yielded montelukast in its acid form, which was further transformed into its dicyclohexylamine salt, which crystallizes in two different polymorphs (Form A and Form B). From said purified and crystalline dicyclohexylamine salt, montelukast in its acid form was recovered by treatment with acid, and then the sodium salt was obtained by treatment of the free acid with a source of sodium ions.

WO 2007/069261 describes a method for the preparation of sodium montelukast. Several intermediate montelukast amine salts are cited, but only dicyclohexylamine salt is exemplified.

Apart from dicyclohexylamine, there have been disclosed several highly voluminous secondary amines useful in the purification step of preparing sodium montelukast. For example, international patent application WO 2006/008751 discloses dipropylamine salt of montelukast.

Additionally, there are disclosures which use primary amines of low molecular weight for the purification of sodium montelukast. However, the process to obtain these amines takes a large number of hours and/or the yields are low. For instance, WO 2006/043846 describes the preparation of pure sodium montelukast by using the tert-butylamine salt of montelukast. As described therein, the tert-butylamine salt of montelukast was obtained after several hours of stirring (32 h) at room temperature.

WO 2007/004237 describes the preparation of alpha-methylbenzylamine salt of montelukast through a process which takes about 24 hours at room temperature.

The present invention provides an improved process for preparing sodium montelukast through a novel crystalline salt of montelukast with a primary amine. Unexpectedly, the process for preparing this novel crystalline salt overcomes the above mentioned problems and allows obtaining highly pure sodium montelukast.

### SUMMARY OF THE INVENTION

As mentioned above, a first aspect of the present invention is cyclopropylamine salt of montelukast in crystalline form.

A second aspect of the invention is related to the process for preparing said cyclopropylamine salt in crystalline form.

A third aspect of the invention is directed to the use of said cyclopropylamine salt in crystalline form for the preparation of sodium montelukast.

A fourth aspect of the invention is directed to a process for preparing sodium montelukast comprising suspending montelukast cyclopropylamine salt in crystalline form in an organic solvent, treating the product thus obtained with a source of sodium ion; and isolating sodium montelukast.

A fifth aspect of the invention is directed to an alternative process for preparing sodium montelukast comprising suspending montelukast cyclopropylamine salt in crystalline form in an organic solvent, treating the resulting suspension with an organic acid, treating the product thus obtained with a source of sodium ion, adding the resulting solution to a second organic solvent; and isolating sodium montelukast.

Also subject-matter of the present invention is a pharmaceutical composition comprising a therapeutically effective amount of cyclopropylamine salt of montelukast in crystalline form as herein defined, together with an appropriate amount of pharmaceutically acceptable excipients or carriers.

Another aspect of the invention relates to the use of the cyclopropylamine salt of montelukast in crystalline form as defined herein for the manufacture of a medicament.

### DESCRIPTION OF THE FIGURES

Figure 1 shows the X-ray powder diffraction pattern (XRPD) of Form II of cyclopropylamine salt of montelukast.
Figure 2 shows the infrared (IR) spectrum of Form II of cyclopropylamine salt of montelukast.
Figure 3 shows the Differential Scanning Calorimetry (DSC) thermogram of form II cyclopropylamine salt of montelukast.
Figure 4 shows the Differential Scanning Calorimetry (DSC) thermogram of form I cyclopropylamine salt of montelukast.

### DESCRIPTION OF THE INVENTION

According to a first aspect, the present invention is directed to a novel cyclopropylamine salt of montelukast which is efficiently and rapidly obtained in crystalline form. Cyclopropylamine salt of montelukast can be easily converted into highly pure sodium montelukast without isolating montelukast free acid and with a good yield.

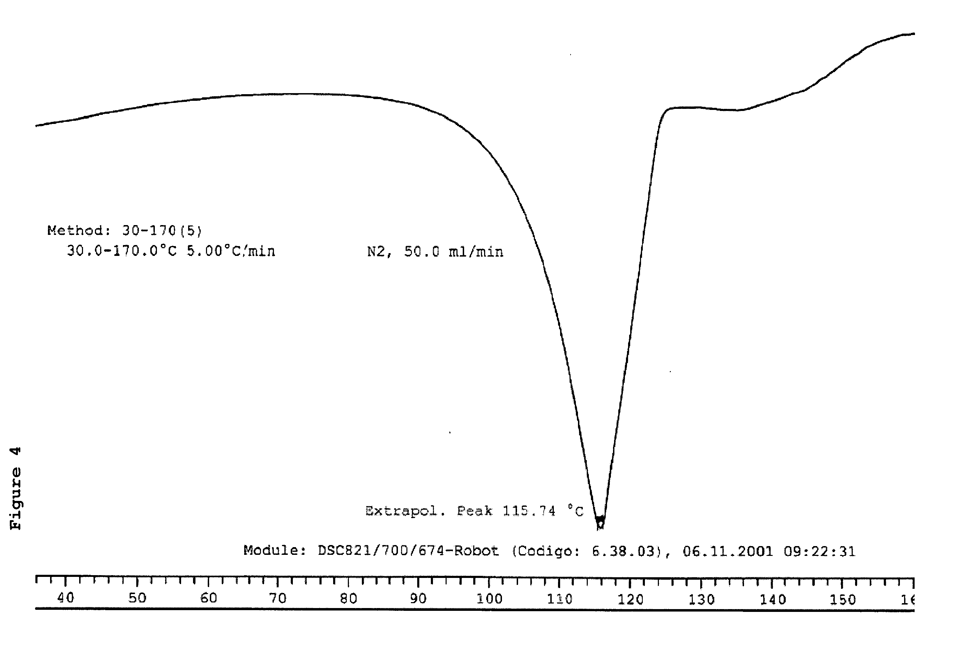

Cyclopropylamine salt of montelukast presents two different crystalline polymorphic forms, named Form I and Form II. Applicants have observed that cyclopropylamine salt of montelukast Form I is not easily obtained although it is stable on storage. Cyclopropylamine salt of montelukast Form I is characterized by the DSC thermogram of Figure 4 with a melting point of 115.74°C.

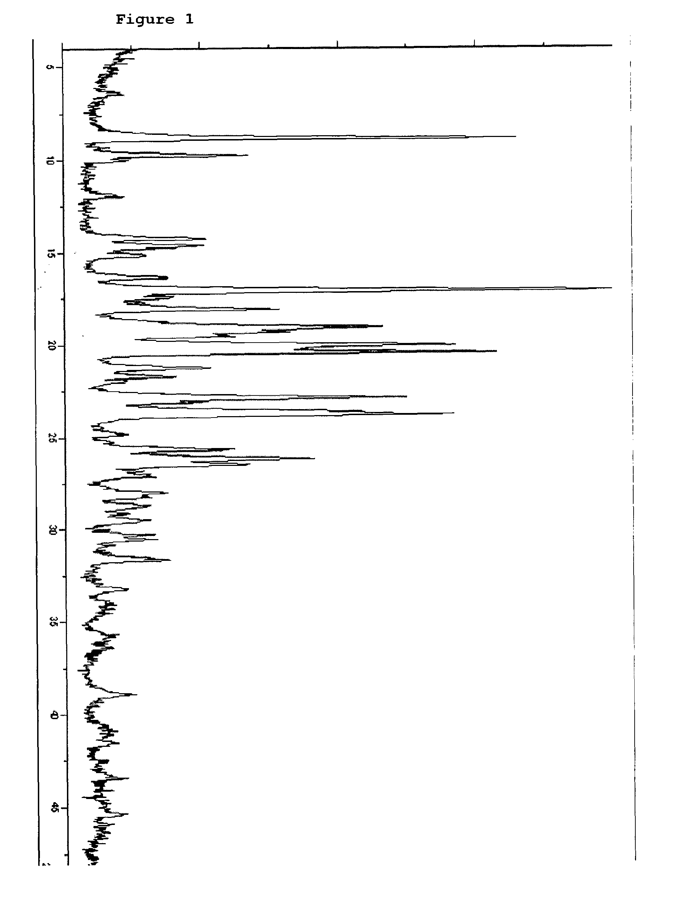

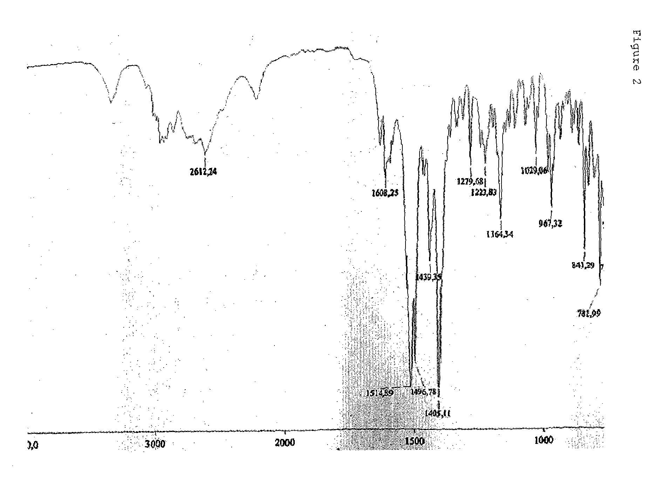

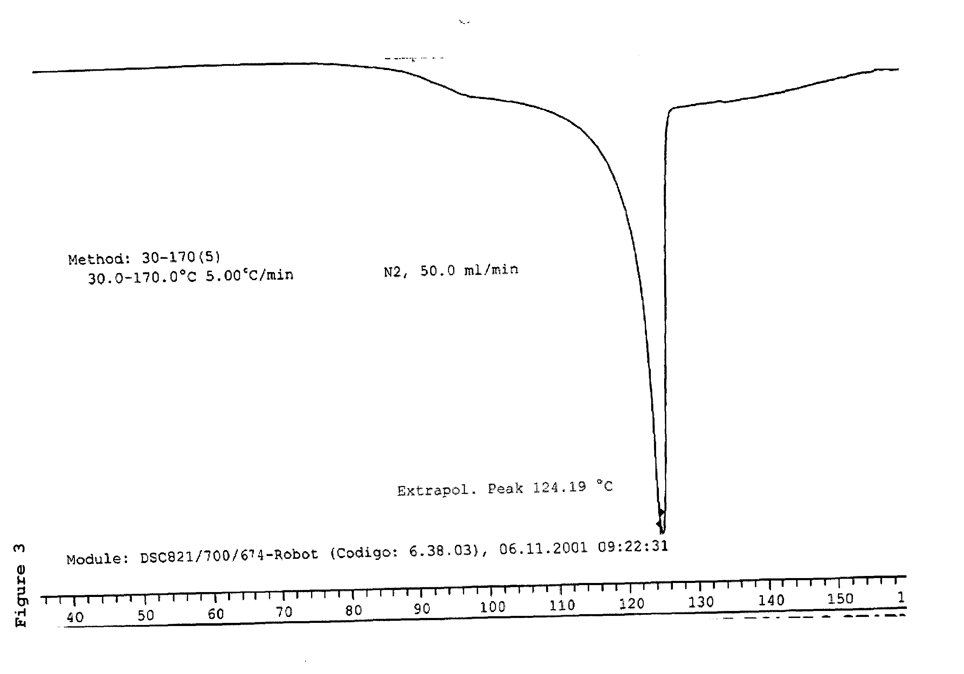

Cyclopropylamine salt of montelukast Form II is thermodynamically stable and is also stable on storage. Form II of cyclopropylamine salt of montelukast is characterized by an X-ray diffraction pattern substantially similar to that presented in Table 1 and in Figure 1, the infrared (IR) spectrum of Figure 2 and DSC thermogram of Figure 3.

**Table 1**

| Pos. [°2Th.] | d-spacing [Å] | Significance |
|---|---|---|
| 8.8521 | 9.98978 | 5.2236 |
| 14.2502 | 6.21543 | 3.0938 |
| 14.6233 | 6.05768 | 2.6794 |
| 15.1419 | 5.85135 | 2.6128 |
| 16.3386 | 5.42538 | 2.2587 |
| 17.0873 | 5.18930 | 9.6880 |
| 18.0715 | 4.90884 | 4.3248 |
| 19.0487 | 4.65917 | 3.9879 |
| 19.5276 | 4.54598 | 2.0866 |
| 20.0214 | 4.43494 | 3.2410 |
| 20.4302 | 4.34713 | 6.0290 |
| 21.2357 | 4.18401 | 2.7052 |
| 22.8788 | 3.88711 | 3.9070 |
| 23.7819 | 3.74151 | 4.5860 |
| 25.6742 | 3.46987 | 2.4356 |
| 26.1770 | 3.40435 | 3.5687 |
| 26.4884 | 3.36504 | 2.2876 |
| 28.6969 | 3.11089 | 2.6474 |
| 29.4746 | 3.03056 | 2.1754 |

XRD pattern was obtained on an analytical X'Pert PRO MPD alpha 1 powder diffractometer, equipped with a CuKα source (λ = 1.54056 Å) and a X'Celerator Detector, operating at 45kV and 40 mA. Each Sample was scanned between 4 and 35° in 2θ, with a step size of 0.017° and a scan rate of 40 s/step.

Differential scanning calorimetry was carried out by means of a Mettler-Toledo DSC-822e calorimeter. Experimental conditions: aluminium crucibles of 40 µl volume, atmosphere of dry nitrogen with 50 ml/min flow rate, heating rate of 5°C/min. The calorimeter was calibrated with indium of 99.99% purity.

FT-IR spectra was recorded on a Perkin Elmer Spectrum One FT-IR spectrophotometer in an ATR accessory, at com-1 resolution, from 650 to 4000 cm-1.

According to the second aspect, the invention provides a method for the preparation of cyclopropylamine salt of montelukast in crystalline form. The process comprises treating montelukast free acid with cyclopropylamine in an organic solvent and isolating cyclopropylamine salt of montelukast. According to a preferred embodiment, the organic solvent is selected from an aromatic hydrocarbon, such as toluene; an ester such as ethyl acetate or isopropyl acetate; an ether such as THF; a ketone such as methylethylketone or a mixture thereof. Preferably, said organic solvent is selected form isopropyl acetate and ethyl acetate.

Montelukast acid used as starting compound for the preparation of the cyclopropylamine salt in crystalline form of the present invention can be prepared according to the method disclosed in European patent application EP07380038.5.

Therefore, in a particular embodiment the method for preparing cyclopropylamine salt of montelukast in crystalline form, further comprises the previous step of reacting 7-chloro-2-vinyl-quinoline and a compound of formula (II) as defined below, in the presence of a palladium based catalyst, 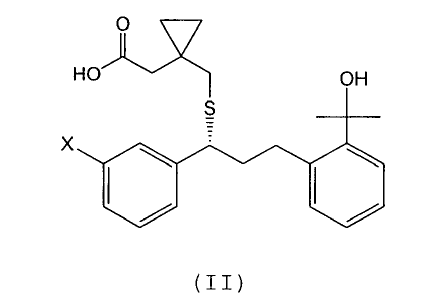 wherein X is a halogen atom or a group of formula -OSO₂R, wherein R is selected from the group consisting of CF₃, tolyl, methyl and F, to obtain Montelukast acid.

Applicants have observed that montelukast acid obtained according to the above process can be used to be converted into the cyclopropylamine salt of the invention either in crystalline form or in solution. Preferably, it is used in solution.

In a particular embodiment, once the cyclopropylamine salt of montelukast is obtained, it is further purified, for example by crystallization or digestion. Election of the most suitable solvent requires only routine experimentation for the skilled person.

Surprisingly, the readily isolable crystalline cycloproplylamine salt, in either form, offers a simple, rapid and efficient method for the preparation of sodium montelukast, resulting in higher yields and fewer impurities.

Another aspect of the present invention relates to the use of the cyclopropylamine salt of montelukast in crystalline form for the preparation of sodium montelukast.

Preferably, sodium montelukast is prepared in amorphous form.

A forth aspect of the invention provides a process for the preparation of sodium salt of montelukast of Formula (I) which comprises suspending the cyclopropylamine salt of montelukast in crystalline form in an organic solvent, treating the product thus obtained with a source of sodium ion and isolating sodium montelukast. Accordingly, the organic solvent may be for example, an aliphatic hydrocarbon, preferably heptane; an aromatic hydrocarbon, preferably toluene; an ester such as ethyl acetate; an ether such as THF; or mixtures thereof. Preferably, the source of sodium ion is selected from NaOH or Sodium tert-Pentoxide. If desired, sodium montelukast can be crystallized according to methods known by a man skilled in the art (such as the methods described in EP 0 737 186 B1).

Particularly, the cyclopropylamine salt of montelukast used for preparing sodium montelukast is obtained by the process mentioned above.

In a preferred embodiment, sodium montelukast is isolated in amorphous form.

According to a particular embodiment, wherein a mixture of solvents is used, the source of sodium is added to a solution of the cyclopropylamine salt in crystalline form with one organic solvent and after stirring, a second organic solvent is added and the resulting suspension is stirred. Preferably, the mixture of solvents used is toluene and heptane.

A fifth aspect of the invention is directed to an alternative process for preparing sodium montelukast through the cyclopropylamine salt in crystalline form. The process comprises a) suspending the cyclopropylamine salt in an organic solvent; b) treating the resulting suspension with an organic acid; c) treating the product thus obtained with a source of sodium ion; d) adding the resulting solution to a second and different organic solvent and e) isolating sodium montelukast. Accordingly, the organic solvent used in both steps a) and d) may be for example, an aliphatic hydrocarbon, preferably heptane; an aromatic hydrocarbon, preferably toluene; an ester such as ethyl acetate; an ether such as THF; or a mixture thereof. In a preferred embodiment the solvent mixture is toluene/heptane. The organic acid is selected from the group of citric acid, acetic acid, oxalic acid, tartaric acid, and the like. Citric acid is preferably used. The source of sodium ion is selected from NaOH or sodium tert-pentoxide. If desired, sodium montelukast can be crystallized according to methods known by a man skilled in the art (such as the methods described in EP 0 737 186 B1).

Particularly, the cyclopropylamine salt of montelukast used for preparing sodium montelukast is obtained by the process mentioned above.

In a preferred embodiment, sodium montelukast is isolated in amorphous form.

Also subject-matter of the present invention is a pharmaceutical composition comprising a therapeutically effective amount of cyclopropylamine salt of montelukast in crystalline form as herein defined, together with appropriate amount of pharmaceutically acceptable excipients or carriers.

As it is said above, it is a part of the present invention the use of the cyclopropylamine salt of montelukast in crystalline form as defined herein for the manufacture of a medicament

The following examples are displayed to illustrate the process of the present invention. They do not intend to limit in any way the scope of the invention defined in the present description.

### EXAMPLES

### Preparation of montelukast acid solution

A mixture of (1-{1-(R)-(3-Bromophenyl)-3-[2-(1-hydroxy-1-methyl-ethyl)-phenyl]-propylsulfanylmethyl}-cyclopropyl)-acetic acid (800 mg, 1.7 mmol), 7-chloro-2-vinylquinoline ( 381 mg, 2.0 mmol), tri(o-tolyl)phosphine (243 mg, 0.8 mmol) and Pd(OAc)₂ (45 mg, 0.2 mmol) in dry DMF (4 mL) is degassed by bubbling nitrogen for 15 minutes. NEt₃ ( 0.7 mL, 5.0 mmol) is added and it is heated at 100 °C for 4 hours. After cooling, it is poured on a mixture of tol/EtOAc 1:1 (50 mL) and water (50 mL). The pH is adjusted to 3-4 with citric acid and the aqueous phase is extracted with more (50 mL) tol/EtOAc 1:1. Then, the solvent is concentrated and the resulting mixture is diluted with either ethylacetate or isopropylacetate.

### Impurities control in products obtained in examples 1 to 6

Most important impurities to be controlled in montelukast salts are:

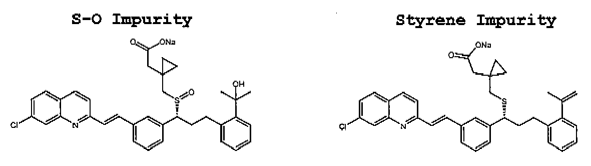

HPLC was performed using the following specifications:
Column: XTerra RP-18, 0.10 m x 4.6 mm x 3.5 µm
Buffer: 0.7 ml H₃PO₄ (85%) to 1 L water
Gradient of Eluent:

| Time (min) | % A |
|---|---|
| 0.00 | 40% |
| 10.00 | 5% |
| 15.00 | 5% |
| 15.01 | 40% |

Stop time: 15 min
Flow: 1 ml / min
Detector: 280 nm
Injection volume: 5 µL
Column temperature: 40°C

### Example 1

### Formation of polymorphic form I of cyclopropylamine salt of montelukast

To 7 ml of stirred, freshly distilled cyclopropylamine, is added, dropwise, 450 mL of a solution of montelukast acid in ethyl acetate (approximately 100 g/L). Once the addition is ended, solution is stirred for 5 minutes, and precipitates a the mixture is stirred for 30 minutes and the suspended solid is filtered, washed with cold ethyl acetate and dried overnight at 45°C in vacuum oven. 39 g of white solid were obtained. **Purity HPLC**: 99,1 %; S-O impurity: 0.12%; Styrene impurity: 0%; Other impurities: 0.77% **1H-NMR (200 MHz, CDCl3),** δ. **(ppm) =** 0.31-0.62 (m, 8H); 1.60(s, 3H, CH3); 1.62(s, 3H, CH3); 2.09-2.71 (m, 7H); 2.85 (m, 1H); 3.2 (m, 1H); 3.59 (t, 1H); 5.10 (s, active H); 7.05-7.24 (m, 3H); 7.28-7.55 (m, 6H); 7.55-7.76 (m, 4H); 8.00-8.16 (m, 2H) **IR (ATR, cm-1)** = 3332, 3052, 2959, 2852, 2606, 2205, 1723, 1702, 1629, 1608, 1582, 1514, 1497, 1461, 1439, 1405, 1358, 1279, 1223, 1164, 1130, 1080, 980, 967, 959, 932, 920, 904, 862, 841, 826, 802, 782, 762, 756, 721, 697, 674. **MS (Acid):** M+1: 586.1 **DSC:** 115.74°C peak

### Example 2

### Formation of polymorphic form II of cyclopropylamine salt of montelukast

To 450 ml of a stirred solution of montelukast acid in isopropyl acetate (approximately 100 g/L) is added, at room temperature, 7 ml of freshly distilled cyclopropylamine. The resulting solution is stirred for 30 minutes and is seeded with 50 mg of pure cyclopropylamine salt of montelukast. Solution is stirred for 1h 30 min. The suspended solid is filtered, washed with cold isopropyl acetate and dried overnight at 45°C in a vacuum oven. 40 g of white solid were obtained.
**Purity HPLC:** 99,7 %;
S-O impurity: 0.07%;
Styrene impurity: 0%;
Other impurities: 0.15% **1H-NMR (200 MHz, CDC13),** δ **(ppm) =** 0.31-0.62 (m, 8H); 1.60(s, 3H, CH3); 1.62(s, 3H, CH3); 2.09-2.71 (m, 7H); 2.85 (m, 1H); 3.2 (m, 1H); 3.59 (t, 1H); 5.10 (s, active H); 7.05-7.24 (m, 3H); 7.28-7.55 (m, 6H); 7.55-7.76 (m, 4H); 8.00-8.16 (m, 2H) **IR (ATR, cm-1)** = 3332, 3052, 2959, 2924, 2852, 2606, 2205,
2145, 1723, 1702, 1629, 1608, 1582, 1514, 1497, 1461, 1439, 1405, 1358, 1279, 1242, 1223, 1164, 1130, 1080, 1056, 1029, 1018, 980, 967, 959, 932, 920, 904, 862, 841, 826, 802, 782, 762, 756, 721, 697, 674.
**MS (Acid):** M+1: 586.2
**DSC:** 124.19°C peak

### Example 3

### Purification of cyclopropylamine salt of montelukast

The 40 g of cyclopropylamine salt of montelukast obtained in Example 2, 180 ml of ethyl acetate and 225 ml of toluene were charged in a clean and dry round bottom flask and stirred at room temperature for 5 hours. Suspended solid is filtered, washed with 180 ml of toluene and 180 ml of hexane and dried overnight at 45°C in a vacuum oven. 37 g of off-white solid were obtained.
**Purity HPLC:** 100 %;
S-O impurity: 0%;
Styrene impurity: 0%;
Other impurities: 0% **¹H-NMR (200 MHz, CDCl₃), δ. (ppm) =** 0.31-0.62 (m, 8H); 1.60(s, 3H, CH₃); 1.62(s, 3H, CH₃); 2.09-2.71 (m, 7H); 2.85 (m, 1H); 3.2 (m, 1H); 3.59 (t, 1H); 5.10 (s, active H); 7.05-7.24 (m, 3H); 7.28-7.55 (m, 6H); 7.55-7.76 (m, 4H); 8.00-8.16 (m, 2H) **¹³C-NMR (200 MHz, CDCl₃), δ. (ppm) =** 6.1, 6.3, 12.1, 12.4,
17.2, 24.1, 30.4, 32.0, 32.3, 39.5, 40.0, 40.9, 41.7, 52.1, 73.9, 119.5, 125.7, 125.8, 127.2, 128.7, 131.8, 135.5, 136.4, 140.3, 144.0, 145.2, 148.5, 157.0, 177.1 **IR (ATR, cm⁻¹) =** 3328, 2958, 2609, 2211, 1629, 1608, 1514, 1496, 1439, 1404, 1279, 1164, 967, 841, 781, 762, 696.
**MS (Acid):** M+1: 586.2
**DSC:** 119.88°C peak, 124.07°C peak

### Example 4

### Preparation of montelukast sodium salt

The 37 g of pure cyclopropylamine salt of montelukast obtained in Example 2 and 187 ml of toluene were charged in a clean and dry round bottom flask under inert conditions. 6 g of sodium tert-pentoxide were added to the suspension, and was stirred for 30' at 40 °C. Solution is added during 5 minutes over 500 ml of cold heptane and is stirred for 1 hour. Suspension is filtered, washed with 2x150 ml of cold heptane and dried overnight at 45°C in a vacuum oven. 33 g of off-white solid were obtained.
**Purity HPLC:** 99.81 %;
S-O impurity: 0.18 %;
Styrene Impurity: 0 %;
Other impurities: 0 % **¹H-NMR (200 MHz, CDCl₃), δ. (ppm) =** 0.15-0.52 (m, 4H); 1.49-1.59 (s, 6H, CH₃); 1.90-2.60 (m, 6H); 2.65 (m, 1H); 3.25 (m, 1H); 3.62 (s, active H); 3.92 (s, 1 H); 6.96-7.21 (m, 3H); 7.26-7.57 (m, 10H); 7.61-7.96 (m, 2H) **¹³C-NMR (200 MHz, CDCl₃), δ. (ppm) =** 12.0, 13.1, 17.3, 31.7, 32.2, 40.0, 44.1, 50.1, 73.2, 119.3, 125.5, 125.6, 127.1, 128.1, 125.6, 128.7, 129.0, 131.5, 135.2, 135.5, 136.0, 136.5, 140.4, 144.0, 145.4, 145.6, 156.8, 180.4 **IR (ATR, cm⁻¹) =** 3347, 3057, 2960, 2926, 1592, 1575, 1496, 1407, 1270, 1241, 1143, 1131, 1068, 1016, 963, 938, 877, 863, 836, 760, 697.
**MS (Acid):** M+1: 586.2
DSC: No defined peaks.

### Example 5

### Preparation of montelukast sodium salt

40 g of pure cyclopropylamine salt of montelukast and 200 ml of heptane were charged in a clean and dry round bottom flask under inert conditions. 6,4 g of sodium tert-pentoxide were added to the suspension, and was stirred for 1 hour at r. t. Solution is filtered, washed with 2x150 ml of cold heptane and dried overnight at 45°C in a vacuum oven. 34 g of off-white solid were obtained.
**Purity HPLC:** 99.85 %;
S-O impurity: 0.08 %;
Styrene impurity: 0 %;
Other impurities: 0.06 % **¹H-NMR (200 MHz, CDCl₃),** δ**. (ppm) =** 0. 15-0. 52 (m, 4H); 1. 49-1.59 (s, 6H, CH₃); 1.90-2.60 (m, 6H); 2.65 (m, 1H); 3.25 (m, 1H); 3.62 (s, active H); 3.92 (s, 1 H); 6.96-7.21 (m, 3H); 7.26-7.57 (m, 10H); 7.61-7.96 (m, 2H) **¹³C-NMR (200 MHz, CDCl₃),** δ. **(ppm) =** 12. 0, 13.1, 17.3, 31. 7, 32.2, 40.0, 44.1, 50.1, 73.2, 119.3, 125.5, 125.6, 127.1, 128.1, 125.6, 128.7, 129.0, 131.5, 135.2, 135.5, 136.0, 136.5, 140.4, 144.0, 145.4, 145.6, 156.8, 180.4 **IR (ATR, cm⁻¹) =** 3347, 3057, 2960, 2926, 1592, 1575, 1496, 1407, 1270, 1241, 1143, 1131, 1068, 1016, 963, 938, 877, 863, 836, 760, 697.
**MS (Acid):** M+1: 586.2
**DSC:** No defined peaks.

### Example 6

### Preparation of montelukast sodium salt

40 g of pure cyclopropylamine salt of montelukast and 160 ml of toluene were charged in a clean and dry round bottom flask under inert conditions. 62 ml of citric acid 0.5 M were added to the suspension dropwise, and was stirred for 30 minutes at r. t. The layers are separated and aqueous layer was extracted with 100 ml of toluene. The combined organic layers were washed with 150 ml of brine and dried over anhydrous magnesium sulphate. Solution was then treated with 6 g of sodium tert-pentoxide for 1 hour at r. t. Solution is then added during 5 minutes over 500 ml of cold heptane and is stirred for 1 hour. Suspension is filtered, washed with 2x150 ml of cold heptane and dried overnight at 45°C in a vacuum oven. 35 g of off-white solid were obtained.
**Purity HPLC:** 99.33 %;
S-O impurity: 0.57 %;
Styrene impurity: 0 %;
Other impurities: 0.09 % **¹H-NMR (200 MHz, CDCl₃), δ. (ppm) =** 0.15-0.52 (m, 4H); 1.49-1.59 (s, 6H, CH₃); 1.90-2.60 (m, 6H); 2.65 (m, 1H); 3.25 (m, 1H); 3.62 (s, active H); 3.92 (s, 1 H); 6.96-7.21 (m, 3H); 7.26-7.57 (m, 10H); 7.61-7.96 (m, 2H) **¹³C-NMR (200 MHz, CDCl₃), δ. (ppm) =** 12.0, 13.1, 17.3, 31.7, 32.2, 40.0, 44.1, 50.1, 73.2, 119.3, 125.5, 125.6, 127.1, 128.1, 125.6, 128.7, 129.0, 131.5, 135.2, 135.5, 136.0, 136.5, 140.4, 144.0, 145.4, 145.6, 156.8, 180.4 **IR (ATR, cm⁻¹) =** 3347, 3057, 2960, 2926, 1592, 1575, 1496, 1407, 1270, 1241, 1143, 1131, 1068, 1016, 963, 938, 877, 863, 836, 760, 697.
**MS (Acid):** M+1: 586.2 **DSC:** No defined peaks.

## Claims

1. Cyclopropylamine salt of montelukast in crystalline form.

2. The salt according to claim 1 in crystalline form II having a X-ray diffraction pattern substantially as shown in Figure 1 and showing the peaks of relative intensity I/I₀ over 20% at the 2θ angles of Table 1.

3. The salt according to claim 2 having an Infrared spectrum substantially as shown in Figure 2.

4. The salt according to claims 2 or 3 having a Differential Scanning Calorimetry trace substantially as shown in Figure 3.

5. The salt according to claim 1 in crystalline form I having a Differential Scanning Calorimetry trace substantially as shown in Figure 4.

6. A process for preparing the salt of montelukast of claim 1 which comprises:
a) treating montelukast free acid with cyclopropylamine in an organic solvent
b) isolating montelukast cyclopropylamine salt in crystalline form.

7. The process according to claim 6 further comprising the previous step of:
reacting 7-chloro-2-vinyl-quinoline and a compound of formula (II) as defined below, in the presence of a palladium based catalyst 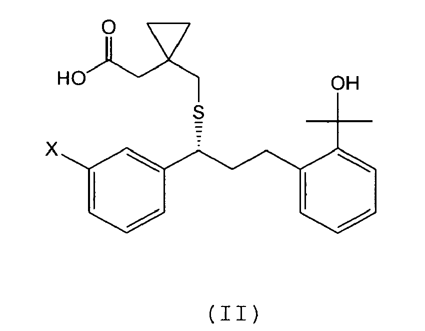
wherein X is a halogen atom or a group of formula - OSO₂R, wherein R is selected from the group consisting of CF₃, tolyl, methyl and F; to prepare montelukast free acid.

8. The process of claim 6 wherein the organic solvent is selected from toluene, THF, methylethylketone isopropylacetate, ethylacetate and mixtures thereof.

9. The process according to claim 8 wherein the organic solvent is isopropylacetate or ethylacetate.

10. The process of claim 6 further comprising the purification of montelukast cyclopropylamine salt.

11. Use of the cyclopropylamine salt of montelukast of claim 1 for the preparation of sodium montelukast.

12. Use according to claim 11 wherein sodium montelukast is obtained in amorphous form.

13. A process for the preparation of sodium montelukast which comprises:
a) suspending the cyclopropylamine salt of montelukast as defined in claim 1 in an organic solvent
b) treating the product thus obtained with a source of sodium ion; and
c) isolating sodium montelukast.

14. The process according to claim 13 wherein the cyclopropylamine salt of montelukast is prepared as defined in any of claims 6 to 10.

15. The process according to claim 13 wherein the organic solvent is selected from toluene, heptane, ethyl acetate, THF and mixtures thereof.

16. The process according to claim 13 wherein the source of sodium ion is NaOH or sodium tert-pentoxide.

17. The process according to claim 13 wherein the sodium montelukast is isolated in amorphous form.

18. A process for the preparation of sodium montelukast which comprises:
a) suspending the cyclopropylamine salt of montelukast as defined in claim 1 in an organic solvent
b) treating the resulting suspension with an organic acid
c) treating the product thus obtained with a source of sodium ion
d) adding the resulting solution to a second organic solvent; and
e) isolating sodium montelukast

19. The process according to claim 18 wherein the cyclopropylamine salt of montelukast is prepared as defined in any of claims 6 to 10.

20. The process according to claim 18 wherein the organic acid is selected from citric acid, acetic acid, oxalic acid and tartaric acid, preferably citric acid.

21. The process according to claim 18 wherein the organic solvent in step a) is toluene, and the organic solvent of step d) is heptane.

22. The process according to claim 18 wherein the source of sodium ion is NaOH or sodium tert-pentoxide.

23. The process according to claim 18 wherein the sodium montelukast is isolated in amorphous form.

24. A pharmaceutical composition comprising a therapeutically effective amount of the cyclopropylamine salt of montelukast as defined in any of the claims 1-5, together with appropriate amount of pharmaceutically acceptable excipients or carriers.

25. Use of the cyclopropylamine salt of montelukast as defined in any of the claims 1-5 for the manufacture of a medicament.
